# EUROPEAN PATENT APPLICATION

(11) **EP 3 428 286 A1**
(43) Date of publication of application: **16.01.2019**
(21) Application number: 17305926.2
(22) Date of filing: 13.07.2017
(51) Int. Cl.: C12Q 1/68

(54) **PREDICTIVE TEST OF ANTI-TNF ALPHA RESPONSE IN PATIENTS WITH AN INFLAMMATORY DISEASE**

(71) Applicant: Université de Bordeaux, 33000 Bordeaux (FR); Centre Hospitalier Universitaire de Bordeaux, 33400 Talence (FR); Institut Polytechnique de Bordeaux, 33400 Talence (FR); Centre National de la Recherche Scientifique, 75016 Paris (FR)
(72) Inventor: SCHAEVERBEKE, Thierry, 33200 Bordeaux (FR); BAZIN, Thomas, 33000 Bordeaux (FR); HOOKS, Katarzyna, 33140 Villenave d'Ornon (FR); NIKOLSKI, Macha, 33000 Bordeaux (FR)
(74) Representative: Novagraaf Technologies

(57) **Abstract**

The present invention relates to an *ex vivo* method for predicting anti-TNF alpha response in a patient with an inflammatory disease in which this treatment is generally indicated, comprising the steps of:
a) Measuring, before any anti-TNF alpha treatment, the level L_{M} of *Burkholderiales* in a patient stool sample, and
b) Calculating the score S1=L_{M}/L_{ref},
Wherein:
• If S1 > 1, the patient is considered likely to have a clinical response to an anti-TNF alpha treatment, or,

If S1 ≤ 1, the patient is considered unlikely to have a clinical response to an anti-TNF alpha treatment.

It also relates to an *ex vivo* method for predicting anti-TNF alpha response in a patient with an inflammatory disease in which this treatment is generally indicated, and to the use of at least one bacteria selected from the group comprising *Burkholderiales, Serratia marcescens, Klebsiella oxytoca, Enterococcus gallinarum, Weissella cibaria* and *Coprococcus eutactus,* as a predictive biomarker of the clinical outcome of an anti-TNF alpha treatment in an inflammatory disease.

## Description

### Technical field

The present invention refers to an *ex vivo* method for predicting anti-TNF alpha response in a patient with an inflammatory disease.

Therefore, the present invention has utility in the medical and pharmaceutical fields.

In the description below, the references into brackets ([ ]) refer to the listing of references situated at the end of the text.

### Background of the Invention

Spondyloarthritis (SpA) is a group of chronic inflammatory diseases that affects axial and/or peripheral joints and sometimes extra-articular organs such as eyes, skin, and gastrointestinal tract. Although its pathophysiology remains imperfectly understood, a genetic background has been identified, characterized by a strong association with the HLA-B27 genotype and a weak link with up to 40 other genes (IL-23R, ERAP1, TNFRSF15...). The role of some environmental factors has also been shown, such as smoking. More recently, a large body of evidence has emphasized the implication of the gut in the pathophysiology of SpA, and more specifically of an intestinal dysbiosis. In the HLA-B27 transgenic murine models, the germ-free animals failed to develop the disease phenotype that was restored by the introduction of bacteria in food supply, specifically with some bacterial cocktails containing *Bacteroidetes* species. In human, overlap between SpA and inflammatory bowel disease (IBD) is frequent: about 5-10% of SpA patients develop IBD, while up to 30% of IBD patients may develop inflammatory arthritis; moreover up to 60% of patients with SpA present microscopic gut inflammation. In addition, clinical remission of SpA is always associated with normal digestive histology, while the persistence of rheumatological symptoms is mostly associated with persistent intestinal inflammation. These elements suggest a close physiopathological link between these two groups of diseases, the recent demonstration of the crucial role of the gut microbiota in IBD raising the same question in SpA.

Three next-generation sequencing (NGS) studies compared the gut microbiota of diseased patients and healthy controls. First, Stoll *et al.* examined gut microbiota of 25 pediatric patients with enthesitis-related arthritis and 13 healthy controls (Stoll ML, Kumar R, Morrow CD, et al. Altered microbiota associated with abnormal humoral immune responses to commensal organisms in enthesitis-related arthritis. Arthritis Res Ther 2014;16:486 ([1**])**). They showed that these patients exhibited a significant reduction of *Faecalibacterium prausnitzii,* as observed in Crohn's disease, a condition often associated with SpA. Second, Costello *et al.* studied terminal ileal biopsies obtained during colonoscopy in nine AS patients and nine controls. They observed an increase in *Lachnospiraceae, Veillonellaceae, Prophyromonadaceae* and *Bacteroidaceae* and a decrease in *Ruminococcaceae* and *Rikenellaceae* in patients (Costello M-E, Ciccia F, Willner D, et al. Intestinal dysbiosis in ankylosing spondylitis. Arthritis Rheumatol Hoboken NJ Published Online First: 21 November 2014 ([2**])**). Third, Tito *et al.* performed investigation of bacterial composition from ileal and colonic biopsies from 27 SpA patients (Tito RY, Cypers H, Joossens M, et al. Dialister as microbial marker of disease activity in spondyloarthritis. Arthritis Rheumatol Hoboken NJ Published Online First: 7 July 2016 ([3**])**). They found different microbial profiles associated with the status of inflammation in the tissue and observed positive correlation between abundance of *Dialister* sp. and Ankylosing Spondylitis Disease Activity Score (ASDAS). Together these three studies indicate that SpA patients exhibit a decrease in the *Clostridiales* order, marked changes in *Bacteroides* genus and decrease in *Verrucomicrobiaceae* family.

Tumour necrosis factor alpha (TNF-α) inhibitors have revolutionized the treatment of spondyloarthritis patients who failed to respond to NSAID and conventional Disease-Modifying Anti-rheumatic Drugs (DMARDs). However, non-response to these treatments is a major concern for clinicians, as only around 30% to 50% of patients exhibit a meaningful clinical response (Moltó A, Paternotte S, Claudepierre P, et al. Effectiveness of tumor necrosis factor α blockers in early axial spondyloarthritis: data from the DESIR cohort. Arthritis Rheumatol Hoboken NJ 2014;66:1734-44 ([4**])**; Maxwell LJ, Zochling J, Boonen A, et al. TNF-alpha inhibitors for ankylosing spondylitis. Cochrane Database Syst Rev 2015;:C0005468 ([5**])**). The mechanism of action of these widely used molecules is still a matter of debate. The most commonly accepted hypothesis is that, by acting on host immune cells, they down-regulate the inflammatory cascade leading to clinical symptoms (Byng-Maddick R, Ehrenstein MR. The impact of biological therapy on regulatory T cells in rheumatoid arthritis. Rheumatology 2015;54:768-75 ([6**])**).

Chronic inflammatory diseases include rheumatology pathologies, such as ankylosing spondylitis, rheumatoid arthritis, juvenile idiopathic arthritis, psoriatic arthritis, and IBDs, such as Crohn's disease and ulcerative colitis. These pathologies are also treated with anti-TNF alpha, and meet the same problems of non-responding patients to this treatment.

Thus, a need exists of method for predicting anti-TNF alpha response in patients with an inflammatory disease, especially inflammatory diseases for which a treatment with anti-TNF alpha is generally indicated and/or for which a marketing authorisation of anti-TNF alpha exists. The present invention fulfills these and other needs.

### Description of the invention

The Applicants has found surprisingly that some modifications of the microbiota composition are observed after 3-month of anti-TNF treatment (M3) of patients with chronic inflammatory disease, especially SpA, but no specific taxon was modified, whatever the clinical response.

They surprisingly showed that a reduced microbial diversity in non-responders patients at enrollment (M0) was rectified after anti-TNF-α treatment, despite the failure of treatment.

The Applicants identified a particular taxonomic node before anti-TNF-α treatment that can predict the clinical response as a biomarker, with a higher proportion of *Burkholderiales* order in future responder patients. A high proportion of *Burkholderiales* was found to be predictive of the clinical response to anti-TNF-α treatment.

He also found that the microbiota composition of non-responders was characterized by a greater instability over time.

Accordingly, in a first aspect, the present invention provides an *ex vivo* method for predicting anti-TNF alpha response in a patient with an inflammatory disease in which this treatment is generally indicated, comprising the steps of:
a) Measuring, before any anti-TNF alpha treatment, the level L_{M} of *Burkholderiales* in a patient stool sample, and
b) Calculating the score S1=L_{M}/L_{ref,}

Wherein:
- If S1 > 1, the patient is considered likely to have a clinical response to an anti-TNF alpha treatment, or,
- If S1 ≤ 1, the patient is considered unlikely to have a clinical response to an anti-TNF alpha treatment.

In other words, the method of the invention makes it possible to establish, before any treatment with an anti-TNF alpha of a patient with an inflammatory disease, whether the condition of a patient can be improved by such a treatment, or whether the condition of the patient is not likely to be improved by the treatment. The improvement of the condition may be for example a decrease of the severity of the disease, especially at M3.

The method of the invention is performed in a patient stool sample. Advantageously, using stool samples is the easiest way to assess gut microbiota, especially in patients who do not suffer from digestive symptoms, and therefore do not require an endoscopy. Moreover, non-invasive ex vivo methods are generally more desirable for developing biomarkers. Collection of bacteria from stools is known in the art. In the case of fecal microbiota collection/analysis, fresh stools may be collected and immediately processed and stored at -80°C for DNA extraction and sequence/quantification as part of a bacterial analysis as further described below.

The measurement of the level L_{M} of *Burkholderiales* may be any measure allowing to quantify the amount of *Burkholderiales* known by the man skilled in the art. It may be the measurement of at least one of *Burkholderiales* DNA, peptides and/or proteins. For example, total DNA can be extracted from stool sample. The protocol may comprise the extraction of total DNA using an extraction step with mechanical disruption.

Advantageously, the step of measurement may comprise a step of hybridisation or amplification of the DNA, peptides and/or proteins material. The amplification may be realized by any method known in the art appropriate for comparing the amount of two sequences, for example quantitative DNA analysis such as polymerase chain reaction (PCR), 16s DNA Sequencing, NGS, culture based methods, flow cytometry, microscopy, microchip hybridization based methods such as immunostaining, and any other means that would be obvious to a person skilled in the art. The polymerase chain reaction may be a 16S DNA amplification, or, preferably, a quantitative polymerase chain reaction (qPCR). qPCR is particularly advantageous as it can also be applied to the detection and precise quantification of DNA in samples to determine the presence and abundance of a particular DNA sequence in these samples.

It is possible to introduce, in the step of measurement, especially in the case of qPCR, a normalization step in order to correct the differences between the compared samples. The normalization step may be realised by any known method known in the art. It may be for example a method comprising normalising the copy number of Burkholderiales per unit of weight of DNA used and copy number of 16S rRNA, or by amplifying with the Burkholderiales primers and universal primers two PCR products, cloning them or cleaning them up from the gel, using them in the qPCR to make a standard curve and then normalising the number of Burkholderiales copies by rRNA number of copies.

Advantageously, the quantitative polymerase chain reaction uses primers that detect at least 92%, preferably at least 95%, for example at least 98% or at least 99%, of *Burkholderiales* species in the patient stool sample. Advantageously, bacteria other than *Burkholderiales* species are detected in an amount less than 5%, preferably less than 3%.

For example, the primers used may be specific of *Burkholderiales* species, or may be derived of primers specific of bacteria that are upstream in the phylogenetic classification, for example Betaproteobacteria. For example, it may be a pair of primers having the following sequences, or of a sequence having at least 90% of identity with the sequences:
Pair N°1: 5'- GGG GAA TTT TGG ACA ATG GG -3' (SEQ ID NO: 1) and
5'- GWA TTA CCG CGG CKG CTG -3' (SEQ ID NO: 2).
Pair N°2: 5'-CCT ACG GGA GGC AGC AG-3' (SEQ ID NO: 3) and
5'-ATT ACC GCG GCT GCT GGC A-3' (SEQ ID NO: 4). This second pair of primers was published by Watanabe et al. (Watanabe et al.: "Design and evaluation of PCR primers to amplify bacterial 16S ribosomal DNA fragments used for community fingerprinting." J. Microbiol. Methods. 44, 253-262 (2001) ([7**])**).

Alternatively, it may be primers designed on conserved regions of *Burkholderiales.*

The number of pairs of primers may be comprised between 1 and 10, for example it may be 1, or 2, or 3 pairs of primers.

In the case where a qPRC is used, it may comprise a step of calculating a ratio between a representative value of the *Burkholderiales* and a representative value of all the bacteria of the body, or at least all the bacteria found in the intestine. These values may be obtained by using at least one set of primers to amplify the *Burkholderiales* and at least one set of primer to amplify all the bacteria of the sample.

The representative value of all bacteria may be obtained by any quantification method known in the art, for example using qPCR with universal bacterial primer such as those of SEQ ID NO: 3 and/or SEQ ID NO: 4.

L_{ref} may be established on a significant patients sample comprising : a group (1) of patients with clinical improvement after treatment with TNF-alpha on the one hand, and group (2) of patients who did not show any clinical improvement after treatment with TNF-alpha on the other. By measuring the level of *Burkholderiales* at M0 in each of these groups, the L_{ref} value can be determined as the mean value separating patients from group (1) of patients in group (2). For example, "significant patients sample" may refer to a sample of about 60 patients, preferably about 90 patients.

According to the invention, *Burkholderiales* may be any bacteria belonging to the Burkholderiales order. It may be at least one bacteria chosen among the families Alcaligenaceae, Burkholderiaceae, Comamonadaceae, Oxalobacteraceae, Ralstoniaceae, and Sutterellaceae. More particularly, it may be *Burkholderiales* that are present in the digestive tract and that are not pathogen. It may be for example a species belonging to the genus *Burkholderia* selected from the group comprising *Burkholderia ambifaria, Burkholderia andropogonis, Burkholderia anthina, Burkholderia brasilensis, Burkholderia caledonica, Burkholderia caribensis, Burkholderia caryophylli, Burkholderia cenocepacia, Burkholderia cepacia, Burkholderia cepacia complex, Burkholderia dolosa, Burkholderia fungorum, Burkholderia gladioli, Burkholderia glathei, Burkholderia glumae, Burkholderia graminis, Burkholderia hospita, Burkholderia kururiensis, Burkholderia mallei, Burkholderia multivorans, Burkholderia phenazinium, Burkholderia phenoliruptrix, Burkholderia phymatum, Burkholderia phytofirmans, Burkholderia plantarii, Burkholderia pseudomallei, Burkholderia pyrrocinia, Burkholderia sacchari, Burkholderia singaporensis, Burkholderia sordidicola, Burkholderia stabilis, Burkholderia terricola, Burkholderia thailandensis, Burkholderia tropica, Burkholderia tuberum, Burkholderia ubonensis, Burkholderia unamae, Burkholderia vietnamiensis and Burkholderia xenovorans.*

Preferably, *Burkholderiales* bacteria are those that are likely to be present in the human bowel.

The inflammatory disease may be any inflammatory disease likely to be improved by anti-TNF alpha treatment. It may be a chronic inflammatory disease. The disease may be selected from the group comprising spondyloarthritis, especially ankylosing spondylitis, psoriasis, rheumatoid polyarthritis, psoriatic arthritis, Crohn's disease, ulcerative colitis and juvenile idiopathic arthritis.

According to the invention, the clinical response predicted by the *ex vivo* method of the invention refers to a level of symptoms as described in disease activity index corresponding to the disease, such as Ankylosing Spondylitis Disease Activity Score (ASDAS) and/or Bath Ankylosing Spondylitis Disease Activity Index (BASDAI) score(s), Psoriasis Area and Severity Index (PASI), Rheumatoid Arthritis Disease Activity Index (RADAI), Disease Activity for Psoriatic Arthritis (DAPSA), Crohn's disease activity index (CDAI), Pediatric Crohn's disease activity index (PCDAI) Harvey-Bradshaw index, Ulcerative colitis activity index (UCAI), Pediatric Ulcerative colitis activity index (PUCAI), Paris classification of pediatric Crohn's disease, Juvenile Disease Activity Score (JADAS), and the like. For example in SpA patients, an absence of clinical response may refer to an ASDAS improvement ≤ 1 at time M3, and a presence of clinical response may refer to an ASDAS improvement ≥ 1.1.

Advantageously, in the case of rheumatic diseases, it may be already known when the method of the invention is performed, that the patient has no clinical response to non-steroidal anti-inflammatory drug (NSAIDs) and/or to disease modifying anti-rheumatic drugs (DMARD).

In a particular embodiment, the method of the invention may further comprise a step of measuring the levels, in the patient stool sample, of at least one bacteria selected from *Serratia marcescens, Klebsiella oxytoca, Enterococcus gallinarum, Weissella cibaria* and *Coprococcus eutactus.* In this embodiment, a high proportion of *Serratia marcescens, Klebsiella oxytoca, Enterococcus gallinarum, Weissella cibaria* at M0 may be predictive of a non-responder patient, and/or a high proportion *Coprococcus eutactus* at M0 may be predictive of a responder patient. In this embodiment, the step of measurement of the at least one bacteria may be realized before, at the same time or after the step of measurement of the level L_{M} of *Burkholderiales.* Advantageously, this is realized at the same time or after. For example, a high proportion may refer to at least 1/10000 of intestinal microbiota. Alternatively, the prediction may be realized by the following steps:
a) Measuring, before any anti-TNF alpha treatment, the level L_{N} of at least one bacteria selected from *Serratia marcescens, Klebsiella oxytoca, Enterococcus gallinarum, Weissella cibaria* and *Coprococcus eutactus,* in the patient stool sample, and
b) Calculating, for each type of bacteria, the score S2=L_{N}/L_{ref2}, Wherein:

### Regarding Coprococcus eutactus, if measured

- If S2 > 1, the patient is considered likely to have a clinical response to an anti-TNF alpha treatment, or,
- If S2 ≤ 1, the patient is considered unlikely to have a clinical response to an anti-TNF alpha treatment;

### Regarding Serratia marcescens, Klebsiella oxytoca, Enterococcus gallinarum and Weissella cibaria, if measured

- If S2 > 1, the patient is considered unlikely to have a clinical response to an anti-TNF alpha treatment, or,
- If S2 ≤ 1, the patient is considered likely to have a clinical response to an anti-TNF alpha treatment.

L_{ref2} can be established, for each of these bacteria, on a significant patients sample comprising: a group (1) of patients with clinical improvement after treatment with TNF-alpha on the one hand, and group (2) of patients who did not show any clinical improvement after treatment with TNF-alpha on the other. By measuring the level of the at least one bacteria, at M0, in each of these groups, the L_{ref2} value can be determined as the mean value separating patients from group (1) of patients in group (2). For example, "significant patients sample" may refer to a sample of about 60 patients, preferably about 90 patients.

The method of the invention may further comprise a step of measuring the level, in the patient stool sample, of genes involved in synthesis of at least one of lipopolysaccharides, ubiquinone and phenylpropanoids. Advantageously, a high proportion, in comparison to a reference value, of genes involved in synthesis of lipopolysaccharides, ubiquinone and phenylpropanoids may be predictive of non-responder patients. The evaluation of genes proportions is known in the art. It may be realized by a PCR, more particularly a qPCR.

According to the invention, the *ex vivo* method for predicting anti-TNF alpha response in a patient with an inflammatory disease may comprise the steps of:
a) Measuring, before any anti-TNF alpha treatment, the alpha diversity index L_{V} of the fecal microbiota in a patient stool sample, and
b) Calculating the microbiota alpha diversity score D1=L_{V}/L_{ref3}, wherein L_{ref3} is a reference value of alpha diversity index,
Wherein:
- If D1 > 1, the patient is considered likely to have a clinical response to an anti-TNF alpha treatment, or,
- If D1 ≤ 1, the patient is considered unlikely to have a clinical response to an anti-TNF alpha treatment.

In a particular embodiment, this method involving the alpha diversity index L_{V} of the fecal microbiota may be realized alone, i.e. without the method involving *Burkholderiales,* as it can be a predictive method for anti-TNF alpha response in a patient with an inflammatory disease as such. Preferably, this method may be performed before, at the same time or after the method involving *Burkholderiales.*

According to the invention, microbiota alpha diversity evaluates within-community diversity, i.e. diversity at the scale of one sample.

The fecal microbiota composition may be analysed by methods known in the art, for example by OTU or taxonomic assignment with tango. It may be expressed by a diversity index, which can be computed for example from the OUT occurrence matrix.

The alpha diversity index is a quantitative measure that reflects how many different types (such as species) there are in a dataset (a community), and simultaneously takes into account how evenly the basic entities (such as individuals) are distributed among those types. Several alpha diversity indexes exist, for example Shannon, Simpson and Sorenson. Preferably, the reference value of alpha diversity index is calculated based on Shannon and/or Simpson index, for example as indicated in Wang et al. (Wang et al. : « Reduced diversity in the early fecal microbiota of infants with atopic eczema », J Allergy Clin Immunol. 2008 Jan;121 (1):129-34 ([8**])**).

L_{ref3} can be established on a significant patients sample comprising: a group (1) of patients with clinical improvement after treatment with TNF-alpha on the one hand, and group (2) of patients who did not show any clinical improvement after treatment with TNF-alpha on the other. By analyzing fecal microbiota at M0 in each of these groups, the L_{ref3} value can be determined as the mean value separating patients from group (1) of patients in group (2). For example, "significant patients sample" may refer to a sample of about 60 patients, preferably about 90 patients.

In any embodiment of the invention, the technical features explained above are applicable.

Another object of the invention relates to the use of at least one bacteria selected from the group comprising *Burkholderiales, Serratia marcescens, Klebsiella oxytoca, Enterococcus gallinarum, Weissella cibaria* and *Coprococcus eutactus,* as a predictive biomarker of the clinical outcome of an anti-TNF alpha treatment in an inflammatory disease. As indicated above, *Burkholderiales* and *Coprococcus eutactus* are predictive biomarkers of a clinical response to an anti-TNF alpha treatment, whereas *Serratia marcescens, Klebsiella oxytoca, Weissella cibaria* and *Enterococcus gallinarum* are predictive biomarkers of an absence of clinical response to an anti-TNF alpha treatment.

Another object of the invention relates to a primer for the sequencing and/or amplification of *Burkholderiales* having the sequence 5'- GGG GAA TTT TGG ACA ATG GG -3' (SEQ ID NO: 1).

The invention is further illustrated by the following examples with regard to the annexed drawings that should not be construed as limiting.

### Brief description of the figures

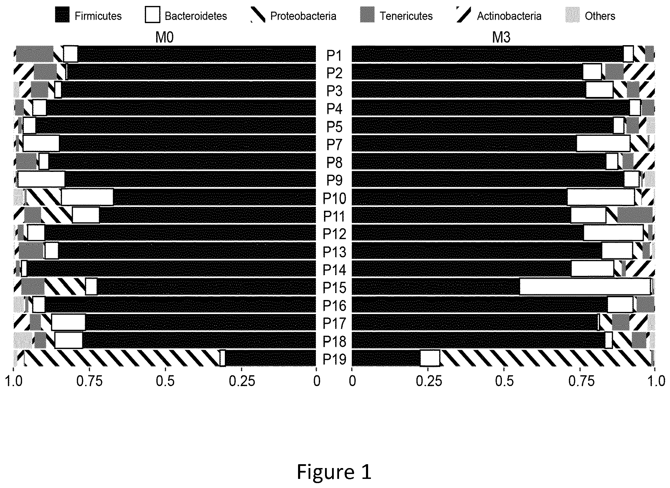

- Figure 1 represents the proportion of reads assigned to different phyla. For each patient reads assigned with Tango were summed up at the phylum level. Proportions of reads belonging to five dominant phyla according to the legend above are shown. Each row corresponds to one patient at M0 and M3, left and right, respectively. P1-P5 responders, P7-P11 partial responders, P12-P19 non responders. Median proportions per phylum ± SD are as follows: Firmicutes (black) - 0.82 ± 0.15, Bacteroides (white) - 0.05 ± 0.08, Tenericutes (dark grey) - 0.03 ± 0.03, Proteobacteria (antislash) 0.02 ± 0.15.

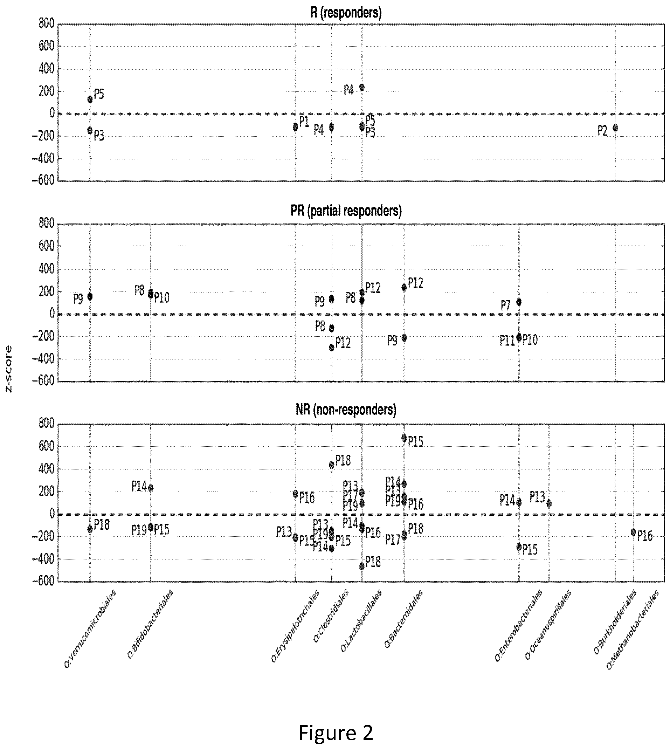

- Figure 2 represents z-scores analysis at the order level. Each point represents z-score between M0 and M3 for one order for one patient. Dots above the zero black dotted represent an increase in the corresponding taxa's proportion in the corresponding patient's gut microbiome after the TNF alpha treatment, while dots below this line represent a decrease. Reads assigned with Tango were summed up for each patient at the order level and normalized. The z-scores were calculated between proportions of reads of each order at M0 and M3 (relative to the total number of reads in the sample) for each patient and filtered by |*z*| > 100. P1-P5 responders, P7-P11 partial responders P12-P19 non responders.

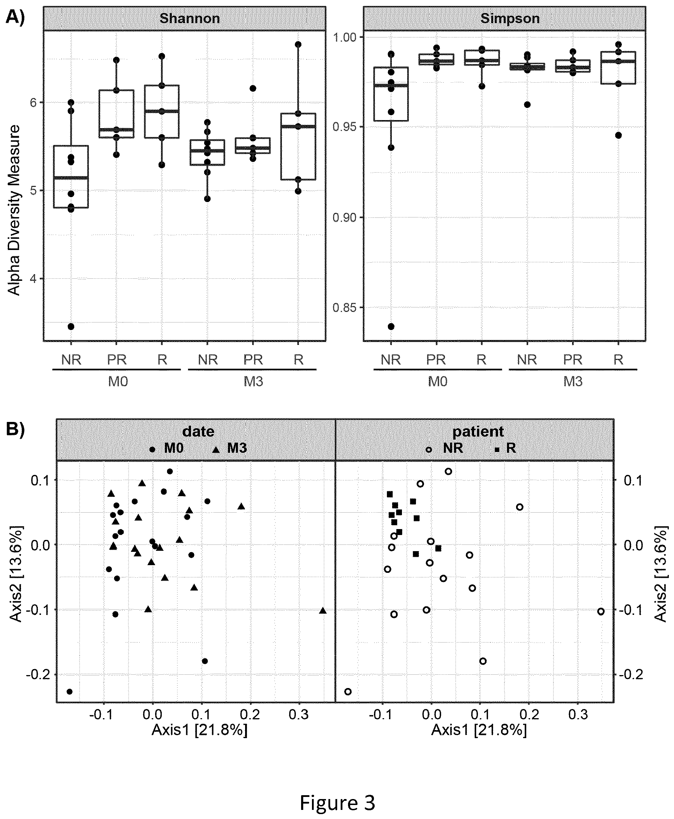

- Figure 3 represents diversity plots for microbiota patient samples. A) Shannon (left) and Simpson (right) diversity indices calculated based on OTU analysis for each type of patient at M0 and M3. Shannon index for NR is significantly different than for R at M0 (two-tailed t-test with unequal variance, p-value = 0.04). B) PCoA plot of β-diversity calculated by weighted UniFrac distances on OTU occurrence table. Timepoints M0 (circle) and M3 (triangle) do not form separate clusters (ANOSIM, R = -0.011, p-value 0.641). Right plot is typed depending of patient's response : Non responders = empty circles; responders = squares (partial responders are removed for clarity). Patients with different level of response form significant clusters (ANOSIM, R = 0.1032, p-value 0.042).

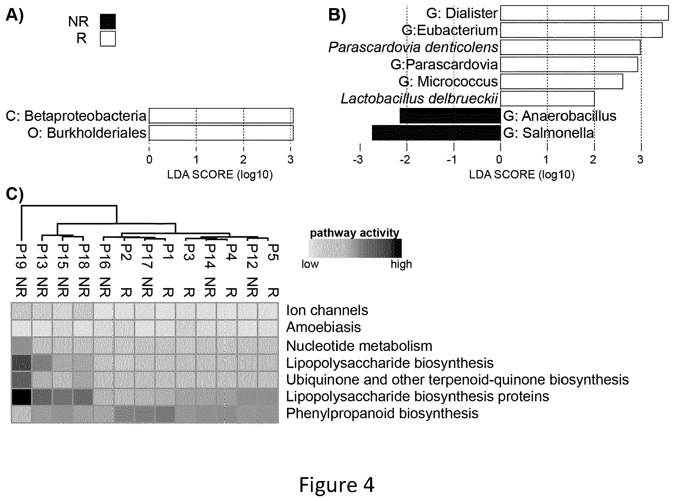

- Figure 4 represents biomarkers of responders and non-responders. LEfSE analysis distinguishing characteristics of taxonomic composition of responders (white) and non-responders (black) at M0 (A) and M3 (B). Biomarkers are coloured according to the response: responders (white) and non-responders (black). Taxa of higher level than species are denoted as follows: G: genus, C: class and O: order. C) Heatmap showing most diversely activated pathways between responders and non-responders at M0 as predicted by PICRUSt (the pathway activity varying from light grey to black).

### Examples

### Example 1:

The aim of this study was to investigate the modification of the intestinal microbiota in patients suffering from SpA three months after the introduction of an anti-TNF-α treatment, and (i) to look for a relationship between the characteristics of the microbiota composition and the clinical response to treatment and (ii) to find taxa correlating with clinical response.

### METHODS

### Study design and patients

A bicentric prospective observational hospital-based exploratory study was conducted. Patients' inclusion criteria were as follows: (i) at least 18 years of age, with a diagnosis of axial only or axial and peripheral spondyloarthritis fulfilling the ASAS criteria, (ii) naïve to anti-TNF-α, justifying the initiation of an anti-TNF-α treatment according to current guidelines (recommendations of the French Society for Rheumatology) and (iii) affiliated to health insurance. Exclusion criteria were as follows: (i) an inflammatory bowel disease, (ii) history of bowel resection or digestive stoma, or taking antibiotics in the three months preceding the stool collection, (iii) contra-indication to anti-TNF-α therapy, (iv) refusal to sign the informed consent or linguistic or cognitive difficulties that did not allow a full understanding of the consent form, (v) pregnancy or breastfeeding, or the refusal to follow an effective contraception method for all the study duration (for women).

Informed consent was sought from study participants. Nineteen patients were recruited. Clinical characteristics of SpA were registered at two times of stool sampling, at enrollment (MO) and after three months (M3); severity was assessed using ASDAS and BASDAI scores (Braun J, Kiltz U, Baraliakos X, et al. Optimisation of rheumatology assessments - the actual situation in axial spondyloarthritis including ankylosing spondylitis. Clin Exp Rheumatol 2014;32:S-96-104 ([9**])**). CRP levels and HLA-B27 status were obtained by systematic blood tests.

The choice of anti-TNF-α drug and dosage has been left to the discretion of the clinicians in accordance with standard practices. Possible combination with other treatments (immunosuppressants, corticosteroids, non steroidal anti-inflammatory drugs) has been left up to the clinicians and recorded in Table 1 below.

**Table 1:**

| **patient** | **Treatment** | **BASDAI1 (/10)** | **HLAB27** | **CRP mg/I** |
|---|---|---|---|---|
| **P1** | | 4,5 | + | 12 |
| **P2** | | 3,8 | - | 9 |
| **P3** | | 5,4 | + | 21 |
| **P4** | Enbrel (etanercept) | 5,2 | + | 10 |
| **P5** | Humira (adalimumab) | 5,0 | + | 46 |
| **P6** | | 3,5 | + | 7 |
| **P7** | | 7,4 | + | 2 |
| **P8** | | 4,0 | + | 2 |
| **P9** | Enbrel (etanercept) | 3,8 | + | 8 |
| **P10** | Enbrel (etanercept) | 3,1 | + | 13 |
| **P11** | Enbrel (etanercept) | 4,0 | | 9 |
| **P12** | Humira (adalimumab) | 7,6 | + | 1 |
| **P13** | | 2,9 | + | 5 |
| **P14** | | 8,2 | + | 2 |
| **P15** | | 4,8 | - | 2 |
| **P16** | | 1,2 | + | 2 |
| **P17** | Remicade (infliximab) | 5,0 | + | 1 |
| **P18** | Enbrel (etanercept) | 6,3 | + | 10 |
| **P19** | Enbrel (etanercept) | 4,9 | + | 6 |

### Sample collection and DNA extraction

Stool samples were collected at M0 and M3 and frozen at -80°C within 24 hours after collection. DNA contained in feces was extracted with Dneasy® Blood & Tissue Kit (Qiagen) according to DNeasy Blood & tissue handbook (Qiagen). In order to optimize the extraction for Gram-positive bacteria, we added a combination of lysostaphin and lysozyme (20mg/ml in lysis solution). We then used silica columns, provided with the previously described kit, to separate DNA from other prokaryotic cells components.

### 16S amplification

16S DNA from 38 samples (19 x 2) was amplified using 2x Phusion GC Master mix and primers 515F and 806R (5'CTTTCCCTACACGACGCTCTTCCGATCTGTGCCAGCMGCCGCGGT AA (SEQ ID NO: 5) and 5'GGAGTTCAGACGTGTGCTCTTCCGATCTGGACTACHVGGGTWTCTA AT (SEQ ID NO: 6), respectively), targeting variable V4 region of 16S bacterial DNA. Maximal expected amplicon length of 347 bp was compatible with direct paired-end MiSeq Illumina sequencing. The DNA was amplified using Master Mix Phusion GC Buffer (New England Biolabs^{®}). PCR conditions were as follows: 30ng of DNA, two primers with final concentration 10µM each, 25µl of Master Mix Phusion GC Buffer, and completion with water leading to a final volume of 50µl. Cycle conditions were as follows: 1 cycle of 98°C, 30s (hot start activation) ; 25 cycles of 98°C,10s (denaturation) / 60°C, 30s (hybridation) / 72°C, 45s (elongation) ; and 72°C during 7 min (final elongation). Then, purification with magnetic beads was performed (Beckman Agencourt^{®}AMPure).

### Library build and sequencing

Resulting libraries were pooled, normalized and denaturated according to Illumina protocol. Samples were then deposited on a MiSeq flowcell 15M and sequenced using the MiSeq Illumina^{®} sequencer at the Genome Transcriptome facility of the University of Bordeaux, generating paired-end reads of 2 x 250 bp. Raw data have been deposited in the ENA sequence read archive (ENA accession number PRJEB19186).

### 16S bioinformatic sequence analysis

Raw reads were quality filtered using the NGS QC toolkit (version 2.3.3) (Patel RK, Jain M. NGS QC Toolkit: a toolkit for quality control of next generation sequencing data. PloS One 2012;7:e30619 ([10**])**) and kept if both of the paired reads passed the filter criteria. The cutoff for quality score was >20 Q30 and >70% of the total read length should have high-quality bases. Possible human contamination was filtered out by mapping the remaining high quality reads against the human genome (*Homo sapiens* alternate assembly HuRef GCF_000002125.1) using BWA (version 0.7.12) (Li H, Durbin R. Fast and accurate short read alignment with Burrows-Wheeler transform. Bioinforma Oxf Engl 2009;25:1754-60 ([11**])**) with default parameters. Possible chimeric sequences were further identified and eliminated using DECIPHER (version 2.14.1) (Wright ES, Yilmaz LS, Noguera DR. DECIPHER, a search-based approach to chimera identification for 16S rRNA sequences. Appl Environ Microbiol2012;78:717-25 ([12**])**). Remaining reads were aligned with BWA against the 16S sequences contained in the GreenGenes database (v13.5) (DeSantis TZ, Hugenholtz P, Larsen N, et al. Greengenes, a chimera-checked 16S rRNA gene database and workbench compatible with ARB. Appl Environ Microbiol 2006;72:5069-72 ([13**])**) keeping all the hits.

Two complementary methods for analyzing microbiota composition were used: operational taxonomic units (OTU) and taxonomic assignment with tango. First remaining reads after filtering were classified using USEARCH_global method of VSEARCH v2.3.0 (Rognes T, Flouri T, Nichols B, et al. VSEARCH: a versatile open source tool for metagenomics. PeerJ 2016;4:e2584 ([14**])**) against 16S OTUs from GreenGenes database with a 97% identity threshold. We have further analysed microbiota richness of our samples by building rarefaction curves based on the OTU computation with the R package vegan (Oksanen J, Blanchet FG, Friendly M, et al. vegan: Community Ecology Package. 2017. https://cran.r-project.org/web/packages/vegan/index.html ([15**])**). Microbiota alpha diversity as expressed by Shannon and Simpson indexes was computed from the OTU occurrence matrix. To calculate phylogenetic beta diversity, we used a weighted UniFrac metric (Lozupone C, Lladser ME, Knights D, et al. UniFrac: an effective distance metric for microbial community comparison. ISME J 2011;5:169-72 ([16**])**) implemented by R package phyloseq (McMurdie PJ, Holmes S. phyloseq: an R package for reproducible interactive analysis and graphics of microbiome census data. PloS One 2013;8:e61217 ([17**])**) by inputting OTU table and GreenGenes OTU tree. Then, to robustly deal with the reads mapped to multiple taxa, we used Tango for taxonomic assignment with *q*-value parameter set to 0.5 (Alonso-Alemany D, Barré A, Beretta S, et al. Further steps in TANGO: improved taxonomic assignment in metagenomics. Bioinforma Oxf Engl 2014;30:17-23 ([18**])**). This approach allows using ambiguous reads by assigning them to higher taxonomic ranks. Consequently, the number of reads assigned to the root was equal to the total number of high quality reads of the sample. Number of reads assigned at every taxonomic node was normalized by the total number of reads in each sample. Normalized counts of all samples were combined in a global occurrence matrix.

### Prediction of bacterial function

Biological function of the assigned microbiota was predicted using predictive functional metagenome PICRUSt method (Langille MGI, Zaneveld J, Caporaso JG, et al. Predictive functional profiling of microbial communities using 16S rRNA marker gene sequences. Nat Biotechnol 2013;31:814-21 ([19**])**). Briefly, OTU table was normalized by copy number using precomputed tables of gene counts from GreenGenes. The mean of weighted nearest sequenced taxon index (NSTI) scores of 0.082 ± 0.018 suggest a good imputation quality. KEGG orthologs prediction was used to identify gene families. In total 328 KEGG pathways were imputed. Pathways with no proportion higher than 90% were removed, leaving 279 pathways. Pathways were analysed using DESeq2, a *p*-value threshold of 5% and ratio change of more than 5% was considered significant.

### Statistical analysis

Nonparametric Wilcoxon-Mann-Whitney test was used to compare quantitative variables between groups. Correlations were calculated using Spearman method. Correction for multiple-testing was performed using Benjamini Hochberg test. LEfSe method was used to discover metagenomic biomarkers (Segata N, Izard J, Waldron L, et al. Metagenomic biomarker discovery and explanation. Genome Biol 2011;12:R60 ([20**])**).

### RESULTS

### Patients

Given the rarefaction curves, we excluded patient P6 since the asymptote is not reached in the M0 sample for this patient (Supplemental Figure 1). Consequently, the analysis of microbiota sequencing was performed for 18 out of 19 patients who participated in the study (Table 1 and Table 2 below).

**Table 2:**

| **Table 2** Demographic and clinical characteristics of patients | | |
|---|---|---|
| Patients N | | 18 |
| Females | | 5 (28%) |
| Age (years)* | | 37±14 |
| Disease duration (years)* | | 2±14 |
| HLA-B27 positive | | 15 (83%) |
| Localisation | | |
| | Axial | 3 (17%) |
| | Axial and | |
| | peripheral | 15 (83%) |
| M0 | | |
| | CRP (mg/L)* | 7 ± 10.7 |
| | ASDAS* | 2.9 ± 0.8 |
| | BASDAI* | 4.9 ± 1.7 |
| M3 | | |
| | CRP (mg/L)* | 2 ± 1.2 |
| | ASDAS* | 1.4 ± 0.9 |
| | BASDAI* | 2.0 ± 2.3 |
| Response at M3 | | |
| | non responder | |
| | (NR) | 8 (44%) |
| | partial responder | |
| | (PR) | 5 (28%) |
| | responder (R) | 5 (28%) |
| | Δ ASDAS* | 1.3 ± 1.2 |

| | | |
|---|---|---|
| *medians ± SD. | | |

None of them has been treated with any of the DMARDs prior to the study and they were considered eligible for anti-TNF-α treatment. Nine patients were referred by Cochin Hospital and nine by Bordeaux Hospital; 13 men and five women presented with axial Ankylosing Spondylitis (N=3) or axial and peripheral Ankylosing Spondylitis (N=15). Fifteen of them were HLA-B27 positive. Prior to the beginning of the study, the median Ankylosing Spondylitis Disease Activity Score (ASDAS) value was 2.9 ± 0.8. Fifteen patients received etanercept, two received adalimumab and one received infliximab. At time M3, eight patients were determined not to have responded to the treatment (ASDAS improvement ≤ 1), whereas five patients exhibited substantial improvement (Δ ASDAS ≥ 1.1) and another five patients exhibited a major positive response (Δ ASDAS ≥ 2).

### Microbiota composition description

The median number of reads per patient was 671,920. After removing singletons, reads were assigned to 24,732 unique OTUs. We used the taxonomic assignment to compare the profiles of patients' microbiota at a phylum-level (Figure 1). The fecal microbiota of most patients was characterized by a very high proportion of *Firmicutes* followed by *Bacteroidetes, Tenericutes* and *Proteobacteria.* Patient 19, who exhibited the most aggravating disease despite treatment as shown by negative ΔASDAS score and increased inflammation confirmed by a higher level erythrocyte sedimentation rate at M3 *vs* M0, (Table 1), exhibited a clearly different microbiota profile with more *Proteobacteria* than other patients at M0 and M3.

### Effect of the anti- TNF-α treatment on microbiota composition

First we compared the global composition of the fecal microbiota at M0 and M3. For all patients as well as for responders or non-responders subgroups, some modifications were observed after treatment, but differences were not significant after multiple-test correction.

Subsequently, we compared the microbiota composition before and after treatment at the taxonomic levels: order, family, genus and species. There were a number of taxa that differ in proportion at the two time-points (Table 3), with four decreasing and 15 increasing at M3. One more time, these differences were not significant after multiple-test correction. In addition, no characteristic for M0 or M3 microbiota composition was observed using LEfSe method.

**Table 3:**

| **Table 3** Taxa significantly changed between samples at M0 and M3 | | | | |
|---|---|---|---|---|
| | **average % of reads** | | **Wilcoxon** | |
| **ORDER** | **M0** | **M3** | ***p*-value** | **FDR** |
| Bdellovibrionales | 4.39*10⁻⁵ | 0 | 0.040 | 0.662 |
| Flavobacteriales | 4.38*10⁻⁴ | 2.26*10⁻³ | 0.050 | 0.662 |

| **FAMILY** | **M0** | **M3** | ***p*-value** | **FDR** |
|---|---|---|---|---|
| Pseudoalteromonadaceae | 9.71*10⁻⁶ | 9.21*10⁻⁴ | 0.009 | 0.767 |
| Nitrospiraceae | 0 | 6.06*10⁻⁵ | 0.040 | 0.767 |
| Acetobacteraceae | 0 | 4.95*10⁻⁵ | 0.040 | 0.767 |
| Flavobacteriaceae | 4.21*10⁻⁴ | 0.002 | 0.050 | 0.767 |

| **GENUS** | **M0** | **M3** | ***p*-value** | **FDR** |
|---|---|---|---|---|
| Proteus | 0.002 | 0.004 | 0.021 | 0.665 |
| Pseudoalteromonas | 9.71*10⁻⁶ | 9.21*10⁻⁴ | 0.009 | 0.665 |
| Rhizobium | 6.13*10⁻⁵ | 0 | 0.019 | 0.665 |
| Nitrospira | 0 | 6.06*10⁻⁵ | 0.040 | 0.665 |
| Afipia | 1.53*10⁻⁴ | 0 | 0.040 | 0.665 |
| Anaerovorax | 1.02*10⁻⁴ | 8.83*10⁻⁴ | 0.043 | 0.665 |

| **SPECIES** | **M0** | **M3** | ***p*-value** | **FDR** |
|---|---|---|---|---|
| *Staphylococcus epidermidis* | 4.36*10⁻⁵ | 2.38*10⁻⁴ | 0.026 | 0.639 |
| *erevibacterium stationis* | 0 | 5.32*10⁻⁴ | 0.002 | 0.639 |
| *Staphylococcus hominis* | 2.90*10⁻⁵ | 2.52*10⁻⁴ | 0.027 | 0.639 |
| *Pediococcus acidilactici* | 0.029 | 0.014 | 0.031 | 0.639 |
| *Clostridium hylemonae* | 1.04*10⁻⁴ | 3.65*10⁻⁴ | 0.032 | 0.639 |
| *Sphingomonas echinoides* | 3.23*10⁻⁵ | 1.69*10⁻⁴ | 0.038 | 0.639 |
| *Bacteroides ovatus* | 0.114 | 0.182 | 0.047 | 0.639 |

Still using taxonomic assignment, we then compared the variation of proportion of each taxa between M0 and M3 for each patient with the mean value of variation in all patients. This comparison is expressed as a z-score, which is the number of standard deviations that separate the value of interest from the mean of all the values. A value below the mean gives a negative z-score, indicating a significant decrease of the taxa when a value above the mean gives a positive z-score, corresponding to a significant increased proportion of the taxa. Ratio-normalized reads assigned with Tango were summed at the order level and z-scores were calculated between M0 and M3 for each patient and filtered by |*z*| > 100. Patients that responded well to treatment had few taxa changing after treatment, and changes were moderate, with most of taxa (6 out of 8) being reduced. Whereas for each of the non-responding patients many bacterial orders exhibited drastic changes (Figure 2), the corresponding taxa increased or decreased chaotically in their proportion. These results suggest that patients who did not respond to anti-TNF-α treatment had high disease activity and unstable microbiota composition.

Finally we analysed the alpha and beta-diversity of the samples. Alpha diversity evaluates within-community diversity, i.e. diversity at the scale of one sample. Beta-diversity compares the composition of different communities, i.e. of different samples. Upon comparing alpha diversity as assessed by Shannon and Simpson indices we observed a difference between responder (R) and nonresponder (NR) patients at M0 (Figure 3A). The clinical response to anti-TNF-α treatment observed for a given patient at M3 correlated with the observed sample diversity at M0 (Spearman r = 0.54, *p*-value = 0.022). This suggests that patients with a reduced initial microbiota diversity are more likely to fail to the anti-TNF-α treatment. However, the treatment abolishes the differences among patient groups as shown by the absence of any differences among the Shannon and Simpson indices calculated at M3, suggesting that, independently to the clinical response, anti-TNF treatment was able to restore the fecal microbial diversity in all patients. There was no relationship between any specific treatment (etanercept, adalimumab and infliximab) or clinical feature of the disease (axial, peripheral, severity...) and microbiota diversity at the beginning or at the end of the study. When phylogenetic beta-diversity, assessed by weighted UniFrac, was used to establish microbiota distances among patients, there was no apparent grouping of patients before and after treatment (Figure 3B, left graph). In contrast, microbiota profiles of responders (M0+M3) appeared to aggregate compared to non-responders (Figure 3B, right graph). Microbiota within responding patients appeared to be more uniform compared to that from non-responding patients, the latter being revealed as a random scattering of the points on the PCoA plot (Figure 3B, right graph).

### Microbial composition can predict response to anti-TNF-a treatment

We focused on the two subgroups of patients - those strongly responding to anti-TNF-α treatment (R, Δ ASDAS ≥ 2) and those showing no response (NR, Δ ASDAS ≤ 1) and we looked for the microbiota composition that can be predictive of the treatment outcome. We found multiple taxa at different levels to be differentially present between R and NR at M0, however after multiple test correction these results became not significant (Table 4).

**Table 4:**

| **Table 4** Significant differentially present taxa between R and NR samples at M0 | | | | |
|---|---|---|---|---|
| | **average % of reads** | | **Wilcoxon** | |
| **ORDER** | **R** | **NR** | ***p*-value** | **FDR** |
| Xanthomonadales | 5.17*10⁻⁴ | 3.09*10⁻⁵ | 0.023 | 0.751 |
| Burkholderiales | 1.526 | 6.03*10⁻⁴ | 0.045 | 0.751 |

| **FAMILY** | **R** | **NR** | ***p*-value** | **FDR** |
|---|---|---|---|---|
| Xanthomonadaceae | 5.17*10⁻⁴ | 0 | 0.023 | 0.800 |
| Carnobacteriaceae | 0.005 | 0.025 | 0.045 | 0.800 |
| Microbacteriaceae | 1.31*10⁻⁵ | 2.94*10⁻⁴ | 0.048 | 0.800 |

| **GENUS** | **R** | **NR** | ***p*-value** | **FDR** |
|---|---|---|---|---|
| Cryocola | 0.001 | 5.14*10⁻⁵ | 0.047 | 0.769 |
| Butyrivibrio | 3.81*10⁻⁴ | 1.09*10⁻⁴ | 0.045 | 0.769 |
| Serratia | 3.25*10⁻⁵ | 0.006 | 0.004 | 0.769 |
| Sutterella | 0.008 | 0.034 | 0.048 | 0.769 |

| **SPECIES** | **R** | **NR** | ***p*-value** | **FDR** |
|---|---|---|---|---|
| *Coprococcus eutactus* | 0.038 | 0.007 | 0.045 | 0.738 |
| *Weissella cibaria* | 0 | 2.42*10⁻⁴ | 0.045 | 0.738 |
| *Serratia marcescens* | 3.25*10⁻⁵ | 0.004 | 0.004 | 0.738 |
| *Bacteroides eggerthii* | 0.059 | 2.01*10⁻⁴ | 0.007 | 0.738 |
| *Klebsiella oxytoca* | 3.25*10⁻⁵ | 0.028 | 0.038 | 0.639 |
| *Bradyrhizobium elkanii* | 0.007 | 3.30*10⁻⁴ | 0.042 | 0.738 |
| *Enterococcus gallinarum* | 0 | 0.002 | 0.045 | 0.738 |

Interestingly, NR patients have higher proportion of pathogenic *Serratia marcescens, Klebsiella oxytoca* that exhibits cytotoxic effects (Joainig MM, Gorkiewicz G, Leitner E, et al. Cytotoxic effects of Klebsiella oxytoca strains isolated from patients with antibiotic-associated hemorrhagic colitis or other diseases caused by infections and from healthy subjects. J Clin Microbiol 2010;48:817-24 ([21**])**), *Enterococcus gallinarum* that can cause serious infections (Reid KC, Cockerill III FR, Patel R. Clinical and epidemiological features of Enterococcus casseliflavus/flavescens and Enterococcus gallinarum bacteremia: a report of 20 cases. Clin Infect Dis Off Publ Infect Dis Soc Am 2001;32:1540-6 ([22**])**) and of the opportunistic pathogen *Weissella cibaria,* whereas R patients have a higher proportion of *Coprococcus eutactus* (which is known to negatively correlate with severity of IBD symptoms (Malinen E, Krogius-Kurikka L, Lyra A, et al. Association of symptoms with gastrointestinal microbiota in irritable bowel syndrome. World J Gastroenterol 2010;16:4532-40 ([23**])**). Using LEfSe analysis, we found two taxonomic nodes that can predict clinical response at M3: Betaproteobacteria class and, belonging to it, Burkholderiales order (Figure 4A).

Similarly, at M3 many taxonomic levels appear to be differentially present when R and NR were compared (Table 5). LEfSe analysis confirmed the results of the Wilcoxon test and indicated genus *Dialister* as the most strongly correlated with the responding patients at M3. Non-responders were characterized by the presence of genus *Salmonella* (Figure 4B). The only species consistently differing between R and NR at both M0 and M3 is *Bacteroides eggerthii.* R patients at M3 also exhibit higher proportion of *Lactobacillus delbrueckii.*

**Table 5:**

| **Table 5** Significantly differentially present taxa between R and NR samples at M3 | | | | |
|---|---|---|---|---|
| | **average % of reads** | | **Wilcoxon** | |
| **FAMILY** | **R** | **NR** | ***p*-value** | **FDR** |
| Aerococcaceae | 9.44*10⁻⁵ | 0.003 | 0.042 | 0.874 |

| **GENUS** | **R** | **NR** | ***p*-value** | **FDR** |
|---|---|---|---|---|
| Micrococcus | 4.91*10⁻⁴ | 5.99*10⁻⁵ | 0.014 | 0.865 |
| Parascardovia | 2.03*10⁻⁴ | 0 | 0.023 | 0.865 |
| Anaerofustis | 2.37*10⁻⁴ | 0.003 | 0.023 | 0.865 |
| Subdoligranulum | 0.009 | 9.69*10⁻⁴ | 0.023 | 0.865 |
| Eubacterium | 3.599 | 0.990 | 0.045 | 0.865 |
| Dialister | 4.004 | 0.990 | 0.045 | 0.865 |
| Salmonella | 0 | 3.20*10⁻⁴ | 0.021 | 0.865 |

| **SPECIES** | **R** | **NR** | ***p*-value** | **FDR** |
|---|---|---|---|---|
| *Lactobacillus* | 0.004 | 7.76*10⁻⁵ | 0.007 | 0.762 |
| *delbrueckii* | | | | |
| *Bacteroides eggerthii* | 0.034 | 1.45*10⁻⁴ | 0.010 | 0.762 |
| *Parascardovia* | 2.03*10⁻⁴ | 0 | 0.023 | 0.762 |
| *denticolens* | | | | |
| *Coprococcus catus* | 0.015 | 0.004 | 0.045 | 0.762 |

### Prediction of bacterial function

Additionally, we used PICRUSt (Langille et al. **([19])**) to infer functions performed by microbiota using KEGG pathways database. This computational analysis allows to predict the metagenome functional content, matching genes of species found in our samples with a database that links gene sequences to metabolic functions. We found that at M0, microbiota of half of NR patients presented higher proportional genes involved in synthesis of lipopolysaccharides, ubiquinone and phenylpropanoids (Figure 4C), that was never found in R patients. Other non-responding patients have a profile of microbiota function very similar to that of responders. Patient 19 with an atypical phylum composition is the most distant compared to other patients also in terms of present microbial pathways.

### DISCUSSION

In this study based on microbiota composition analysis before and three months after treatment with TNF-α inhibitors, no specific taxon was observed to be consistently modified by the treatment in all patients. Each of NR patients displayed drastic differences before and after treatment in microbiota composition at order level, whereas R patients displayed only few mild differences, suggesting a higher stability of microbiota composition in R patients. Alpha-diversity of non-responders was lower at M0 compared to two other groups and this difference was rectified after anti-TNF alpha treatment. High proportion of Burkholderiales at M0 was associated with the clinical response at M3, as high proportion of *Dialistersp.* at M3. *Bacteroides eggerthii* was found at higher concentrations in NR patients at both time points, even if the difference was not significant after correction for multiple-testing. All these results suggest possible biomarkers for anti-TNF-α efficacy in SpA patients.

### Strength and weakness of this study

We estimated intestinal microbiota by fecal microbiota analysis, even if the two are not equivalent (Hong P-Y, Croix JA, Greenberg E, et al. Pyrosequencing-Based Analysis of the Mucosal Microbiota in Healthy Individuals Reveals Ubiquitous Bacterial Groups and Micro-Heterogeneity. PLoS ONE 2011 ;6 ([24**])**. However, using stool samples is the easiest way to assess gut microbiota, especially in patients who do not suffer from digestive symptoms, and therefore do not require an endoscopy. Moreover, non-invasive methods are more desirable for developing biomarkers.

Our study is based on 16S rDNA sequencing, which allows only a partial view of microbiota focused on bacteria. Virobiota, mycobiota and eucaryota of intestinal tract are therefore not considered in this study, and dynamic interactions between all these components are consequently not encompassed, although many studies have shown implications of these actors in IBD physiopathology in particular and in human health in general (Clemente JC, Ursell LK, Parfrey LW, et al. The impact of the gut microbiota on human health: an integrative view. Cell 2012;148:1258-70 ([25**])**). Moreover, the quantification of taxonomic nodes is only relative since we use a ratio between the number of reads of a given taxonomic node and the total number of bacterial reads. Moreover, 16S rDNA analysis is not discriminant for close species. Quantitative PCR could allow more precise inter-sample comparison. Although our predictive metabolic functional analysis is computational, it predicts the abundance of gene families with quantifiable uncertainty (Langille **([19])**).

We have not specifically screened inflammatory digestive manifestations in our population, so we have not evaluated the overlap between rheumatologic and digestive clinical manifestations, which could influence the results. We also did not take into account differing diets of patients.

### Impact of TNF-α inhibitors on microbiota

In this study we have shown no significant modification of particular taxa after treatment, but diversity seemed to be restored at M3 in NR.

Modifications of microbiota composition by TNF-α inhibitors could be caused either by indirect or direct effects. These treatments are well-known to heal and profoundly down-regulate inflammation in the wounded digestive mucosa, therefore restoring normal structure of digestive epithelium and control and tolerance functions toward mucosal microbiota. Thus, they could indirectly change microbiota composition.

More precisely, etanercept exerts a specific action on the host, which could be explained by its structure. Etanercept is a recombinant TNF receptor-Fc fusion protein. Etanercept inhibits not only TNF-α but also soluble TNF-ß, aka lymphotoxin-α, while infliximab and adalimumab are monoclonal antibodies that are exclusively directed against TNF-α. Soluble form of lymphotoxin-α controls IgA induction in the lamina propria, and through this process controls microbiota composition. Thus, the effects of etanercept could modify gut microbiota composition in patients *via* reduced IgA levels, which might explain the absence of clinical efficacy in IBD, together with differences in terms of pharmacokinetic and complement dependent cytotoxicity.

Direct action on the intestinal microbiota, *via* an inter-reigns regulation (inter-kingdom interaction), is suggested by several studies. Particularly, bacterial adrenergic receptors have been recently identified in pathogens, allowing a host neuroendocrine hormones sensing and fitness adaptation depending on host condition. The existence of membrane-bound bacterial receptor to TNF-α has been anciently suspected, especially on gram negative bacteria; in this study the presence of TNF-α seemed to increase virulence of *Shigella flexneri. In vitro* studies are needed to test the effects of TNF-α inhibitors used in clinical practice on bacterial gut commensals.

### Characteristics of microbiota as biomarkers of response to treatment

One of our more surprising results was that microbiota composition could predict clinical response to anti-TNF. It could be particularly relevant in SpA treatment to have a test that could predict at baseline which treatment will be the most effective, as different therapeutic options exist. For instance, anti-IL17 drugs, a new option in treatment of AS, could modify microbiota composition differently from what anti-TNF alpha do, as they specifically block the secretion of IL17 by CD4+ T effector cells (Th17), which play a major role in intestinal homeostasis. Experiments should be conducted in order to identify differential effects of these treatments on microbiota composition, and to look for microbial biomarkers at baseline that could predict their efficiency.

Interestingly, such predictive capacity has been emphasized in multiple studies concerning various diseases. Overall diversity as well as presence or absence of specific taxa have been shown to be biomarkers of disease or response to treatment in other disorders. For example, intestinal microbiota has been proposed as a prognosis factor in colorectal cancer. In advanced stages of colorectal cancer an increased colonic colonization by cyclomodulin-producing *E. coli* and enterotoxigenic *B*. *fragilis* and *F*. *nucleatum* has been reported, suggesting a potential use of microbiota as a colorectal cancer prognosis biomarker. Moreover, it has been suggested that chemotherapy toxicity could be dependent on microbiota composition. Indeed, microbial-produced β-glucuronidases modulate irinotecan digestive toxicity. In case of digestive tumors an intact gut microbiota is needed to obtain an optimal response to oxaliplatin. Indeed, microbiota together with the immune system augment intra-tumor oxaliplatin damages, modulating tumor oxidative microenvironment (lida N, Dzutsev A, Stewart CA, et al. Commensal Bacteria Control Cancer Response to Therapy by Modulating the Tumor Microenvironment. Science 2013;342:967-70 ([26**])**). In melanoma, microbiota composition can predict resistance to immunotherapy-induced colitis. Moreover, the response to anti-CTLA4 therapy has been shown to be conditional upon the presence of distinct *Bacteroidetes* species in the intestinal microbiota. In this study the authors shown that *B*. *thetaiotaomicron* or *B*. *fragilis* induced specific T-cell responses that were associated with the anti-CTLA4 therapy efficacy, and that antibiotic-treated or GF mice's tumors did not respond to this treatment. In mice, commensal *Bifidobacterium* genus is associated with spontaneous antitumour immunity effects against melanoma, and acts in synergy with anti PD-L1 therapy. Another recent study in ulcerative colitis patients treated by anti-TNF therapy revealed lower dysbiosis indices and higher abundance of *Faecalibacterium prausnitzii* in responders compared with non-responders at baseline. Furthermore, the authors showed that responders and non-responders exhibited distinct mucosal antimicrobial peptides expression patterns. Moreover, a recent study has shown that low concentrations of *F*. *prausnitzii* are correlated with early recurrence of CD after anti-TNF-α treatment interruption. Studies in the field of rheumatology have shown that rheumatoid arthritis patients have altered gut and mouth microbiome that is partly normalized after DMARDs treatment and could predict response to treatment. However, the effect on the gut microbiome was shown to be moderate compared to the oral microbiome. Patients that responded well to treatment were characterized by a greater number of virulence factors before treatment and also by the reduction in *Holdemania filiformis* and *Bacteroides* sp. after treatment.

### Comparison with previous results

Similarly to previous approaches, that did not find big differences in gut microbiota composition before and after treatment, in our cohort we only observed moderate changes with limited statistical significance. However, those differences are consistent with the previous studies of microbiota in spondyloarthritis. First, we observed an increase of *Proteobacteria* in patient 19. This phylum has a low abundance in gut flora of healthy subjects and its increase have been associated with gastric bypass, metabolic disorders, inflammation and cancer, which is consistent with our observation of unresolved inflammation in this patient. Second, we observed higher proportions of pathogenic and potentially pathogenic species in NR patients, specifically *Klebsiella oxytoca* and *Salmonella* sp. It has been shown before that AS patient produce higher levels of anti-*Klebsiella* and anti-*Enterobacter* secretory IgA, which have been hypothesized to interact with self-antigen HLA B27 and promote disease progression. It is also known that some gastrointestinal (*Salmonella* sp., *Shigella* sp., *Yersinia* sp. and *Campylobacter* sp.) and urethral infections (*Chlamydia* sp.) can trigger reactive arthritis and up to 20% of those cases will develop AS within 10-20 years. Considering that NR patients are characterized by the presence of *Salmonella* sp., especially at M3, we hypothesize that this might have been a contributing factor in their SpA development. Third, previous reports indicated the changes in Bacteroides associated with the state of the disease. The direction of the changes varies depending on the cohort (Costello et al. **([2]);** Tito et al. **([3])**). In our study we observed that all NR patients showed a change in Bacteroides order: five of them had an increase and two a decrease, whereas R patients were not affected. This result suggests that the proportion of this bacterial order varies significantly in rheumatoid conditions.

Interestingly, R patients at M3 also exhibit higher proportion of *Lactobacillus delbrueckii,* species that carries out the fermentation of kefirs and has been previously proposed as probiotic in treatment for IBD.

Magnusson et *al.* showed that abundance of *F. prausnitzii* increased during induction therapy by anti-TNF-α in R, and not in NR. A recent study in Crohn's disease has shown changes in the microbiota composition after TNF-α inhibitor (adalimumab) treatment, with recovery of phylogroups (*Firmicutes, Bacteroides* and *Actinobacteria)* and decrease of *E*. *coli* during treatment. We didn't found comparable results in our study, but our population and treatment molecules were different.

We observed microbiota composition instability over time in NR. Major shifts in microbiota composition have been associated with diseases such as IBD and neurodevelopmental disorders, but never with spondyloarthritis.

### Perspectives

It is now clearly established that subclinical or even symptomatic gut inflammation is associated with SA; nevertheless relationships between cause and effect remain to be established. In the same way, we have only observed an association between microbiota composition clusters and clinical response, without presuming causality. Nonetheless, these results suggest that a specific fecal microbiota signature could be predictive of a good clinical response to the anti-TNF-α treatment, for which no biomarkers currently exist. It could be particularly clinically relevant to have a reliable test before the initiation of this type of treatment, to first avoid a delay in symptom relief, and second to ease the financial burden on health services.

The stability of microbiota composition is considered to be critical for human health in general, and results from a competitive equilibrium within microbiota's diverse bacterial, fungal and viral components. Microbiota stability in patients could be a good prognostic factor in itself. This hypothesis would require longitudinal long-term studies in order to be confirmed.

### References

1 Stoll ML, Kumar R, Morrow CD, et al. Altered microbiota associated with abnormal humoral immune responses to commensal organisms in enthesitis-related arthritis. Arthritis Res Ther 2014;16:486. doi:10.1186/s13075-014-0486-0.
2 Costello M-E, Ciccia F, Willner D, et al. Intestinal dysbiosis in ankylosing spondylitis. Arthritis Rheumatol Hoboken NJ Published Online First: 21 November 2014. doi:10.1002/art.38967.
3 Tito RY, Cypers H, Joossens M, et al. Dialister as microbial marker of disease activity in spondyloarthritis. Arthritis Rheumatol Hoboken NJ Published Online First: 7 July 2016. doi:10.1002/art.39802.
4 Moltó A, Paternotte S, Claudepierre P, et al. Effectiveness of tumor necrosis factor α blockers in early axial spondyloarthritis: data from the DESIR cohort. Arthritis Rheumatol Hoboken NJ 2014;66:1734-44. doi:10.1002/art.38613.
5 Maxwell LJ, Zochling J, Boonen A, et al. TNF-alpha inhibitors for ankylosing spondylitis. Cochrane Database Syst Rev 2015;:CD005468. doi:10.1002/14651858.CD005468.pub2.
6 Byng-Maddick R, Ehrenstein MR. The impact of biological therapy on regulatory T cells in rheumatoid arthritis. Rheumatology 2015;54:768-75. doi :10.1093/rheumatology/keu487.
7 Watanabe et al.: "Design and evaluation of PCR primers to amplify bacterial 16S ribosomal DNA fragments used for community fingerprinting." J. Microbiol. Methods. 44, 253-262 (2001).
8 Wang et al. : « Reduced diversity in the early fecal microbiota of infants with atopic eczema », J Allergy Clin Immunol. 2008 Jan;121(1):129-34.
9 Braun J, Kiltz U, Baraliakos X, et al. Optimisation of rheumatology assessments - the actual situation in axial spondyloarthritis including ankylosing spondylitis. Clin Exp Rheumatol 2014;32:S-96-104.
10 Patel RK, Jain M. NGS QC Toolkit: a toolkit for quality control of next generation sequencing data. PloS One 2012;7:e30619. doi:10.1371 /journal.pone.0030619
11 Li H, Durbin R. Fast and accurate short read alignment with Burrows-Wheeler transform. Bioinforma Oxf Engl 2009;25:1754-60. doi:10.1093/bioinformatics/btp324
12 Wright ES, Yilmaz LS, Noguera DR. DECIPHER, a search-based approach to chimera identification for 16S rRNA sequences. Appl Environ Microbiol 2012;78:717-25. doi:10.1128/AEM.06516-11
13 DeSantis TZ, Hugenholtz P, Larsen N, et al. Greengenes, a chimera-checked 16S rRNA gene database and workbench compatible with ARB. Appl Environ Microbiol 2006;72:5069-72. doi:10.1128/AEM.03006-05
14 Rognes T, Flouri T, Nichols B, et al. VSEARCH: a versatile open source tool for metagenomics. PeerJ 2016;4:e2584. doi:10.7717/peerj.2584
15 Oksanen J, Blanchet FG, Friendly M, et al. vegan: Community Ecology Package. 2017. https://cran.r-project.org/web/packages/vegan/index.html (accessed 10 Mar 2017).
16 Lozupone C, Lladser ME, Knights D, et al. UniFrac: an effective distance metric for microbial community comparison. ISME J 2011;5:169-72. doi:10.1038/ismej.2010.133
17 McMurdie PJ, Holmes S. phyloseq: an R package for reproducible interactive analysis and graphics of microbiome census data. PloS One 2013;8:e61217. doi:10.1371 /journal.pone.0061217
18 Alonso-Alemany D, Barré A, Beretta S, et al. Further steps in TANGO: improved taxonomic assignment in metagenomics. Bioinforma Oxf Engl 2014;30:17-23. doi:10.1093/bioinformatics/btt256
19 Langille MGI, Zaneveld J, Caporaso JG, et al. Predictive functional profiling of microbial communities using 16S rRNA marker gene sequences. Nat Biotechnol 2013;31:814-21. doi:10.1038/nbt.2676
20 Segata N, Izard J, Waldron L, et al. Metagenomic biomarker discovery and explanation. Genome Biol 2011;12:R60. doi:10.1186/gb-2011-12-6-r60
21 Joainig MM, Gorkiewicz G, Leitner E, et al. Cytotoxic effects of Klebsiella oxytoca strains isolated from patients with antibiotic-associated hemorrhagic colitis or other diseases caused by infections and from healthy subjects. J Clin Microbiol 2010;48:817-24*.*
22 Reid KC, Cockerill III FR, Patel R. Clinical and epidemiological features of Enterococcus casseliflavus/flavescens and Enterococcus gallinarum bacteremia: a report of 20 cases. Clin Infect Dis Off Publ Infect Dis Soc Am 2001;32:1540-6 ([22**])**).
23 Malinen E, Krogius-Kurikka L, Lyra A, et al. Association of symptoms with gastrointestinal microbiota in irritable bowel syndrome. World J Gastroenterol 2010;16:4532-40.
24 Hong P-Y, Croix JA, Greenberg E, et al. Pyrosequencing-Based Analysis of the Mucosal Microbiota in Healthy Individuals Reveals Ubiquitous Bacterial Groups and Micro-Heterogeneity. PLoS ONE 2011;6.
25 Clemente JC, Ursell LK, Parfrey LW, et al. The impact of the gut microbiota on human health: an integrative view. Cell 2012;148:1258-70.
26 lida N, Dzutsev A, Stewart CA, et al. Commensal Bacteria Control Cancer Response to Therapy by Modulating the Tumor Microenvironment. Science 2013;342:967-70.

SEQUENCE LISTING

<110>  UNIVERSITE DE BORDEAUX
       INSTITUT POLYTECHNIQUE DE BORDEAUX
       CENTRE NATIONAL DE LA RECHERCHE SCIENTIFIQUE
       CENTRE HOSPITALIER UNIVERSITAIRE DE BORDEAUX

<120>  PREDICTIVE TEST OF ANTI-TNF ALPHA RESPONSE IN PATIENTS WITH AN
       INFLAMMATORY DISEASE

<130>  BNT222773EP00

<160>  6

<170>  PatentIn version 3.5

<210>  1
<211>  20
<212>  DNA
<213>  Artificial Sequence

<220>
<223>  Primer

<400>  1
ggggaatttt ggacaatggg                                                   20


<210>  2
<211>  18
<212>  DNA
<213>  Artificial Sequence

<220>
<223>  Primer

<400>  2
gwattaccgc ggckgctg                                                     18


<210>  3
<211>  17
<212>  DNA
<213>  Artificial Sequence

<220>
<223>  Primer

<400>  3
cctacgggag gcagcag                                                      17


<210>  4
<211>  19
<212>  DNA
<213>  Artificial Sequence

<220>
<223>  Primer

<400>  4
attaccgcgg ctgctggca                                                    19

<210>  5
<211>  47
<212>  DNA
<213>  Artificial Sequence

<220>
<223>  Primer

<400>  5
ctttccctac acgacgctct tccgatctgt gccagcmgcc gcggtaa                    47


<210>  6
<211>  48
<212>  DNA
<213>  Artificial Sequence

<220>
<223>  Primer

<400>  6
ggagttcaga cgtgtgctct tccgatctgg actachvggg twtctaat                   48

## Claims

1. An *ex vivo* method for predicting anti-TNF alpha response in a patient with an inflammatory disease in which this treatment is generally indicated, comprising the steps of:
a) Measuring, before any anti-TNF alpha treatment, the level L_{M} of *Burkholderiales* in a patient stool sample, and
b) Calculating the score S1=L_{M}/L_{ref,}
Wherein:
● If S1 > 1, the patient is considered likely to have a clinical response to an anti-TNF alpha treatment, or,
● If S1 ≤ 1, the patient is considered unlikely to have a clinical response to an anti-TNF alpha treatment.

2. A method according to claim 1, wherein the measure of step a) comprises a quantitative polymerase chain reaction or a hybridisation reaction.

3. A method according to claim 2, further comprising, when a quantitative polymerase chain reaction is realized, a step of calculating a ratio between a representative value of the *Burkholderiales* and a representative value of all the bacteria of the body, wherein the representative values are obtained by using at least one set of primers to amplify the *Burkholderiales* and at least one set of primer to amplify all the bacteria.

4. A method according to claim 3, wherein the quantitative polymerase chain reaction uses primers that detect at least 92% of *Burkholderiales* species in the patient stool sample.

5. A method according to anyone of the preceding claims, further comprising a step of measuring the levels, in the patient stool sample, of at least one bacteria selected from *Serratia marcescens, Klebsiella oxytoca, Enterococcus gallinarum, Weissella cibaria* and *Coprococcus eutactus.*

6. An *ex vivo* method for predicting anti-TNF alpha response in a patient with an inflammatory disease in which this treatment is generally indicated, comprising the steps of:
c) Measuring, before any anti-TNF alpha treatment, the alpha diversity index Lv of the fecal microbiota in a patient stool sample, and
d) Calculating the alpha diversity score D1=L_{V}/L_{ref3}, wherein L_{ref3} is a reference value of alpha diversity index,
Wherein:
If D1 > 1, the patient is considered likely to have a clinical response to an anti-TNF alpha treatment, or,
If D1 ≤ 1, the patient is considered unlikely to have a clinical response to an anti-TNF alpha treatment.

7. A method according to claim 6, which is realized before or after the method as defined in anyone of claims 1 to 5.

8. A method according to anyone of the preceding claims, wherein said patient has no clinical response to non-steroidal anti-inflammatory drug (NSAIDs) and/or to disease modifying anti-rheumatic drugs (DMARD).

9. A method according to anyone of the preceding claims, wherein said inflammatory disease is selected from the group comprising spondyloarthritis, psoriasis, rheumatoid polyarthritis, psoriatic arthritis, Crohn's disease, ulcerative colitis and juvenile idiopathic arthritis.

10. A method according to anyone of the preceding claims, wherein said inflammatory disease is spondyloarthritis.

11. A method according to anyone of the preceding claims, wherein said spondyloarthritis is ankylosing spondylitis.

12. Use of at least one bacteria selected from the group comprising *Burkholderiales, Serratia marcescens, Klebsiella oxytoca, Enterococcus gallinarum, Weissella cibaria* and *Coprococcus eutactus,* as a predictive biomarker of the clinical outcome of an anti-TNF alpha treatment in an inflammatory disease.

13. Use according to claim 12, wherein *Burkholderiales, Weissella cibaria* and *Coprococcus eutactus* are predictive biomarkers of a clinical response to an anti-TNF alpha treatment.

14. Use according to claim 13, wherein *Serratia marcescens, Klebsiella oxytoca* and *Enterococcus gallinarum* are predictive biomarkers of an absence of clinical response to an anti-TNF alpha treatment.

15. Use according to anyone of claims 12 or 14, wherein said inflammatory disease is selected from the group comprising spondyloarthritis, psoriasis, rheumatoid polyarthritis, psoriatic arthritis, Crohn's disease and ulcerative colitis.

16. Use according to claim 15, wherein said inflammatory disease is spondyloarthritis.

17. A primer for the sequencing and/or amplification of *Burkholderiales* having the sequence 5'- GGG GAA TTT TGG ACA ATG GG -3' (SEQ ID NO: 1).
